(19) Europäisches Patentamt
European Patent Office
Office européen des brevets

(11) Veröffentlichungsnummer: **0 002 030**
**B1**

(12) # EUROPÄISCHE PATENTSCHRIFT

(45) Veröffentlichungstag der Patentschrift: **30.09.81**

(21) Anmeldenummer: **78101331.3**

(22) Anmeldetag: **08.11.78**

(51) Int. Cl.³: **C 07 D 235/20,**
C 07 D 263/56,
D 06 L 3/12, // C 11 D 3/42

(54) **Neue fluorhaltige Benz-azol-Derivate, Verfahren zu ihrer Herstellung und ihre Verwendung als optische Aufheller.**

(30) Priorität: **15.11.77 DE 2750947**

(43) Veröffentlichungstag der Anmeldung:
**30.05.79 Patentblatt 79/11**

(45) Bekanntmachung des Hinweises auf die Patenterteilung:
**30.09.81 Patentblatt 81/39**

(84) Benannte Vertragsstaaten:
**BE CH DE FR GB NL**

(56) Entgegenhaltungen:
**DE - A - 1 670 890**
**DE - A - 1 719 353**
**DE - A - 2 645 301**
**DE - B - 1 282 592**
**DE - B - 1 301 791**

(73) Patentinhaber: **HOECHST AKTIENGESELLSCHAFT**
**Zentrale Patentabteilung**
**Postfach 80 03 20**
**D-6230 Frankfurt**
**Main 80 (DE)**

(72) Erfinder: **Pintschovius, Ulrich, Dr.**
**Bad Sodener Strasse 6**
**D-6234 Hattersheim am Main (DE)**
Erfinder: **Schinzel, Erich, Dr.**
**Ostpreussentstrasse 43**
**D-6238 Hofheim am Taunus (DE)**
Erfinder: **Rösch, Günter**
**Hohlweg 17**
**D-6232 Bad Soden am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 030 B1

## Neue fluorhaltige Benz-azol-Derivate, Verfahren zu ihrer Herstellung und ihre Verwendung als optische Aufheller

Die Verwendung von solchen 1,4 - Bis - [benzazolyl] - naphthalinen, die keine fluorhaltigen Substituenten enthalten, ist bereits bekannt, insbesondere aus der deutschen Offenlegungsschrift 1 670 890 und der deutschen Auslegeschrift 1 282 592. Es wurde nun gefunden, daß man im Vergleich zu diesen nichtfluorhaltigen Verbindungen höhere Weißgrade beim optischen Aufhellen erreichen kann, wenn man in diese 1,4 - Bis - [benzazolyl] - naphthaline fluorhaltige Substituenten einführt.

Gegenstand der Erfindung sind somit Verbindungen der allgemeinen Formel

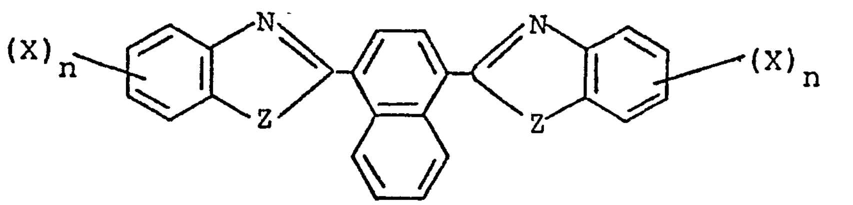

I

in welcher X eine Trifluormethylgruppe oder ein Fluoratom, n 1 oder 2, Z ein Sauerstoffatom oder eine NR-Gruppierung bedeutet, in der R Wasserstoff oder eine $C_1$—$C_4$-Alkylgruppe ist, wobei die Benzoringe durch ein oder zwei $C_1$—$C_4$-Alkyl-, $C_1$—$C_4$-Alkoxy-, Halogen-, Cyan-, Carbo-($C_1$—$C_4$)-alkoxy-, Phenyl- oder Benzylgruppen substituiert sein können, Verfahren zu deren Herstellung und deren Verwendung also optische Aufheller.

Die erfindungsgemäßen Benzoxazolyl-Verbindungen (Z = O) können nach dem im folgenden erläuterten Herstellungs-Verfahren synthetisiert werden, wobei X und n die für die Formel I angegebene Bedeutung haben.

Das Dichlorid der Naphthalin - 1,4 - dicarbonsäure IV wird mit wenigstens 2 Mol o - Amino - phenolen der allgemeinen Formel

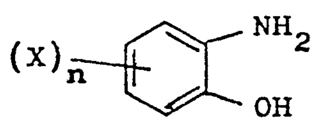

V

vorzugsweise in Gegenwart einer Base kondensiert und die erhaltenen Bis - acylamino - Verbindungen der allgemeinen Formel

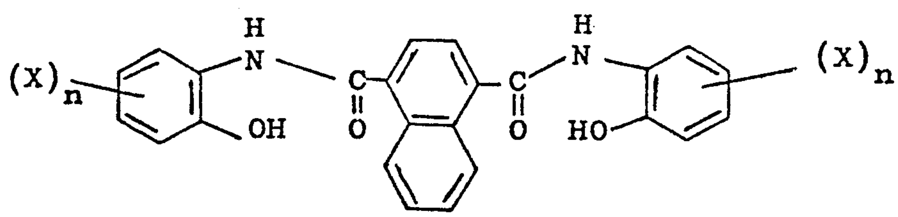

VI

in inerter Gasatmosphäre in hochsiedenden Lösungsmitteln un in Gegenwart von Katalysatoren zu den erfindungsgemäßen Verbindungen (I, Z=O) cyclisiert.

Als o-Aminophenole der Formel V können beispielsweise eingesetzt werden:

3 - Trifluormethyl - 2 - amino - phenol, 4 - Trifluormethyl - 2 - amino - phenol, 5 - Trifluormethyl - 2 - amino - phenol, 6 - Trifluormethyl - 2 - amino - phenol, 3,5 - Di - (trifluormethyl) - 2 - amino - phenol, 4 - Fluor - 2 - amino - phenol, 5 - Fluor - 2 - amino - phenol, 4,6 - Difluor - 2 - amino - phenol, 4 - Methyl - 5 - fluor - 2 - amino - phenol, 5 - methyl - 4 - fluor - 2 - amino - phenol.

Als hochsiedende Lösungsmittel für die Ringschlußreaktion kommen beispielsweise in Betracht: 1,2,4 - Trichlorbenzol, Trichlorbenzol-Gemische, Chlornaphthaline, Tetralin oder Methylnaphthalin-Gemische. Die Reaktionstemperatur für die Cyclisierung liegt bei 150 bis 260°C, vorzugsweise 200 bis 230°C. Als Ringschlußkatalysatoren können Säuren, einschließlich Lewis-Säuren dienen, wie z.B. Zinkchlorid, p-Toluolsulfosäure oder Borsäure.

Die erfindungsgemäßen Benzimidazol-Verbindungen (Z = NR) können nach dem im folgenden erläuterten Herstellungsverfahren gewonnen werden, wobei X, R und n die unter I angegebene Bedeutung besitzen. Das Dichlorid der Naphthalin - 1,4 - dicarbonsäure IV wird mit mindestens 2 Mol o-Nitroanilinen der allgemeinen Formel

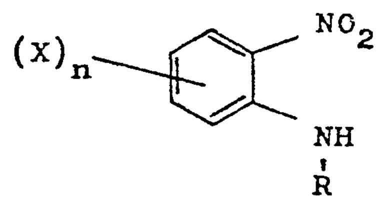

VII

vorzugsweise in Gegenwart einer Base kondensiert, die erhaltenen Bis - nitro - acylamino - Verbindungen der allgemeinen Formel

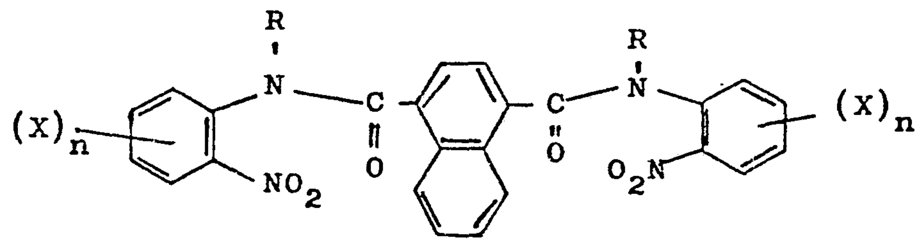

VIII

werden nach bekannten Verfahren katalytisch mit Wasserstoff oder einem anderen Reduktionsmittel in einem geeigneten Lösungsmittel zu den entsprechenden Bis - o - aminoacylaminen

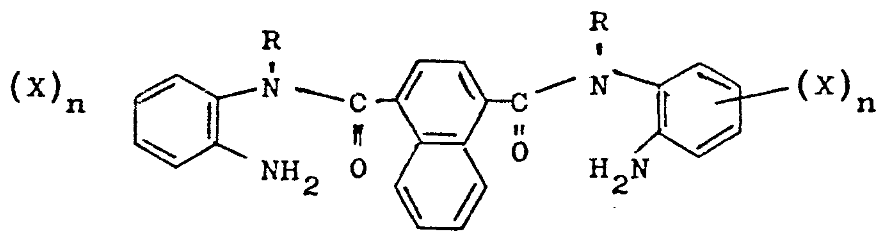

IX

reduziert und letztere werden dann durch Einwirkung von Säuren zu den erfindungsgemäßen Verbindungen (Z = NR) cyclisiert. Diese Ringschlußreaktion erfolgt bei Temperaturen von etwa 100—120°C in einem inerten organischen Lösungsmittel wie etwa Benzol, Toluol, Chlorbenzol in Gegenwart Säuren wie Toluolsulfonsäure. Bevorzugt geschieht die Cyclisierung in siedendem Eisessig.

Enthalten die Ausgangsverbindungen der Formel VII in para-Stellung zur Nitrogruppe eine elektronegative Gruppe, so ist es besser, dieses o-Nitroanilin zuerst zum entsprechenden o-Phenylendiamin zu reduzieren und dann erst mit dem Naphthalin - 1,4 - dicarbonsäurechlorid umzusetzen bzw. zu cyclisieren, wie oben beschrieben. In der oben geschilderten Reaktionsfolge wird die Gruppe R durch Verwendung eines N-substituierten o-Nitroanilins eingeführt. Daneben ist es auch möglich, diese Gruppe erst nach der Cyclisierung durch eine entsprechende Alkylierung am Stickstoffatom nach bekannten Verfahren einzuführen.

Die Acylierung der o-Aminophenole der Formel IV und der o-Nitroaniline der Formel VII erfolgt im allgemeinen unter den gleichen Bedingungen bei Temperaturen bis ca. 100°C, vorzugsweise bei 70—80°C in einem inerten organischen Lösungsmittel wie etwa Benzol, Dioxan, Toluol, Dichloräthan oder Toluol in Gegenwart einer Base etwa eines Trialkylamins.

Als o - Nitro - aniline der allgemeinen Formel VII können beispielsweise eingesetzt werden: 3 - Trifluormethyl - 2 - nitro - anilin, 4 - Trifluormethyl - 2 - nitro - anilin, 5 - Trifluormethyl - 2 - nitro - anilin, 3,5 - Bis - (trifluormethyl) - 2 - nitro - anilin, 4 - Fluor - 2 - nitro - anilin, 4,6 - Difluor - 2 - nitro - anilin, 4 - Trifluormethyl - 2 - nitromethylanilin, 4 - Trifluormethyl - 2 - nitro - äthylanilin.

Die neuen erfindungsgemäßen Verbindungen haben aufgrund ihres Fluoreszenzvermögens ein weites Anwendungsgebiet. Vor allem dienen sie zum optischen Aufhellen der verschiedensten natürlichen und synthetischen organischen Materialien. Darunter sind auch solche organische Materialien zu verstehen, die zur Veredlung mineralischer Stoffe, beispielsweise anorganischer Pigmente, verwendet werden können.

Als aufzuhellende Substrate seien beispielsweise folgende Materialien genannt: Synthetische Fasern, wie z.B. solche aus Acetylcellulose, Polyester vie z.B. Polyäthylenterephthalat, Polyamiden, Polyolefinen, Polyvinylchlorid, Polyvinylidenchlorid und Polyacrylnitril, sowie Folien, Filme, Bänder und Formkörper aus solchen Materialien.

Die erfindungsgemäßen Verbindungen können gelöst in organischen Lösungsmitteln zum Einsatz kommen oder in wäßriger (saurer) Lösung oder Dispersion, vorteilhaft unter Zuhilfenahme eines

Dispergierungsmittels, eingesetzt werden.

Als Dispergierungsmittel kommen beispielsweise Seifen, Polyglykoläther, die sich von Fettalkoholen, Fettaminen oder Alkylphenolen ableiten. Cellulosesulfitablaugen oder Kondensationsprodukte von gegebenenfalls alkylierten Naphthalinsulfonsäuren mit Formaldehyd sowie Polyvinylalkohole in Frage.

Die Aufhellung des Fasermaterials mit der wäßrigen oder eventuell organischen Aufhellerflotte erfolgt entweder im Ausziehverfahren bei Temperaturen von vorzugsweise etwa 20 bis 150°C oder unter Thermosolierbedingungen, wobei das Textilmaterial beispielsweise mit der Aufhellerlösung bzw. -dispersion imprägniert oder besprüht und beispielsweise zwischen Walzen auf einen Restfeuchtigkeitsgehalt von etwa 50 bis etwa 120% abgequetscht wird. Anschließend wird das Textilmaterial etwa 10 bis etwa 300 Sekunden einer Temperaturbehandlung, vorzugsweise mittels Trockenhitze, bei etwa 120 bis etwa 240°C unterzogen. Dieser Thermosolierprozeß kann auch mit anderen Ausrüstungsoperationen, z.B. der Ausrüstung mit Kunstharz zum Zwecke der Pflegeleichtigkeit, kombiniert werden, wobei gegebenenfalls nach der Imprägnierung und Trocknung bei 100—150°C das Material 5—20 Minuten bei 150—200°C zum Zwecke der Vernetzung kondensiert wird.

Die Verbindungen der allgemeinen Formel (I) können auch Waschmitteln zugesetzt werden. Diese können die üblichen Füll- und Hilfsstoffe, wie Alkalisilikate, Alkaliphosphate bzw. kondensierte Phosphate, Alkaliborate, Alkalisalze der Carboxymethylcellulose, Schaumstabilisatoren, wie Alkanolamide höherer Fettsäuren oder Komplexbildner wie lösliche Salze der Äthylendiamintetraessigsäure oder Diäthylentriaminpentaessigsäure, sowie chemische Bleichmittel, wie Perborate oder Percarbonate, Perborataktivatoren von Typ der Polyessigsäureamide, die in Verbindung mit den Perverbindungen zur Abspaltung von Peressigsäure führen, und Desinfektionsmittel enthalten.

Ferner können die erfindungsgemäßen Verbindungen hochmolekularen organischen Materialien vor bzw. während deren Verformung zugesetzt werden. So kann man sie beispielsweise bei der Herstellung von Fasern, Filmen, Folien, Bändern, Platten oder anderen Formkörpern den Kunststoffpulvern, thermoplastischen Massen, Schmelzen, Polymerlösungen oder Dispersionen beifügen, z.B. vor dem Verspinnen in der Spinnmasse lösen. Geeignete Verbindungen können auch vor der Polykondensation oder Polymerisation, wie im Falle von Polyamid-6, Polyamid-6,6 oder linearen Polyestern vom Typ des Polyäthylenterephthalats den niedermolekularen Ausgangsmaterialien zugesetzt werden.

Auf Polyacrylnitril und Polyacrylnitril-Mischpolymerisaten werden mit den erfindungsgemäßen Verbindungen besonders hohe Weißgrade erhalten, wenn sie in Gegenwart von Bleichmitteln wie z.B. Natriumchlorit im sauren Bereich bei pH 2—5 eingesetzt werden. Ein besonderer Vorteil der Verfahrensprodukte ist die geringe Beeinflussung der Aufhelleffekte bei Änderung des pH-Wertes in dem genannten Bereich.

Die Menge der erfindungsgemäß zu verwendenden Verbindungen der allgemeinen Formel, bezogen auf das optisch aufzuhellende Material, kann je nach Einsatzgebiet und gewünschtem Effekt in weiten Grenzen schwanken. Sie dann durch einfache Vorversuche leicht ermittelt werden und liegt im allgemeinen zwischen etwa 0,01 und etwa 2%, vorzugsweise zwischen 0,02—0,2% bezogen auf das aufzuhellende Material.

Die folgenden Beispiele erläutern die Erfindung näher. Die Temperaturen sind in Celsius-Graden angegeben. Prozentangaben sind Gewichtsteile, sofern nichts anderes angegeben ist.

Tabelle 1

Verbindung der Formel

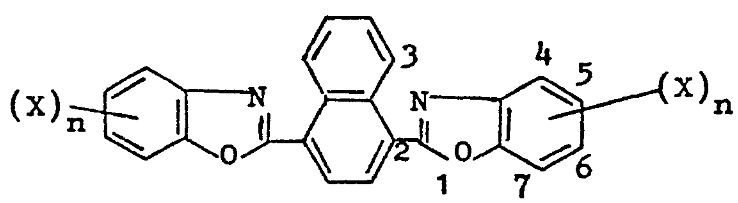

| Nr. | Substituent n | X | Pos | Schmp. | Absorption in DMF-Lg. λ [nm] | ε | Fluoreszenz in DMF-Lg. |
|---|---|---|---|---|---|---|---|
| 101 | 1 | $CF_3$ | 4 | 275—276,5° | 376 | 33 100 | bläul. viol. |
| 102 | 1 | $CF_3$ | 5 | 212—214° | 372 | 31 600 | violett |
| 103 | 1 | $CF_3$ | 6 | 221—222° | 373 | 31 600 | bläul. viol. |
| 104 | 2 | $(CF_3)_2$ | 4,6 | 201—203° | 378 | 30 300 | blau |
| 105 | 1 | F | 5 | 228—230° | 376 | 32 600 | bläul. viol. |

106 1,4 - Bis - (5 - methyl - 6 - fluorbenzoxazolyl - 2) - naphthalin; Schmp. 266—268°C, Absorption in DMF: λ 378 nm, ε 36 300; Fluoreszenz bläul. violett.

Tabelle 2

Verbindung der Formel

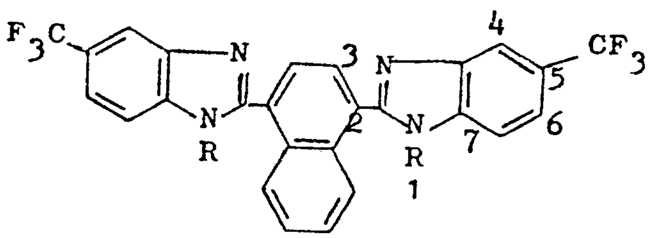

| Nr. | Substituent R | Schmp. | Absorption in DMF-Lg. λ [nm] | ε | Fluoreszenz in DMF-Lg. |
|---|---|---|---|---|---|
| 110 | H | 317—318° | 346 | 28 600 | bläul. viol. |
| 111 | $C_2H_5$ | 297—298° | 304 | 20 800 | violett |
| 112 | $CH_3$ | 351—352° | 310 | 22 700 | violett |

Beispiel 1

*Die Herstellung der Verbindung 102 (Tabelle 1) wird folgendermaßen durchgeführt:*

Aus 21,6 g Naphthalin-1,4-dicarbonsäure und 28 g Thionylchlorid wird in 78 g Toluol das Dichlorid hergestellt. Nach Entfernung des überschüssigen Thionylchlorids läßt man die Säurechloridlösung bei 80° zu einer Lösung von 34,8 g 4 - Trifluormethyl - 2 - amino - phenol, 24,4 ml N,N-Dimethylanilin und 80 ml Dioxan tropfen und rührt 5 Stunden bei dieser Temperatur nach. Das Reaktionsgemisch wird mit Wasserdampf destilliert, der Niederschlag abgesaugt und mit verd. Salzsäure und Wasser gewaschen. Nach dem Trocknen erhält man 53,1 g Acylaminoverbindung vom Schmp. (Zers.) 262 bis 264°C.

53 g der vorstehend genannten Acylaminoverbindung werden in einer Stickstoffatmosphäre in 300 ml Trichlorbenzol in Gegenwart von 0,18 g p-Toluolsulfosäure 75 Min. auf 200 bis 215° erhitzt, wobei das abgespaltene Wasser mit wenig Trichlorbenzol übergeht. Nachdem die Hauptmente des Lösungsmittels (ca. 200 ml) abdestilliert ist, werden 300 ml Methanol zugesetzt. Durch Absaugen, Waschen mit Methanol und Trocknen erhält man 31,8 g Benzoxazol (102) als gelbes Kristallpulver.

Nach Umkristallisation aus Toluol und DMF liegt der Schmp. bei 212 bis 214°.

In gleicher Weise können die Verbindungen 101, 103, 104, 105 der Tabelle 1 synthetisiert werden.

Beispiel 2

*Herstellung von 1,4-Bis-[5-trifluormethyl-benzimidazolyl-(2)]-naphthalin (Verb. 110)*

Eine Lösung von 25,3 g Naphthalin-1,4-dicarbonsäurechlorid in 90 ml Toluol läßt man unter Rühren bei 80° zu einer unter Stickstoff auf 70° vorgewärmten Mischung von 42 g 4 - Trifluormethyl - 2 - nitro - anilin, 26 ml N,N-Dimethylanilin und 100 ml Dioxan zulaufen.

Nach 5 Stunden Rühren bei 80° wird das Reaktionsgemisch mit Wasserdampf destilliert, die Acylamino-Verbindung im Rückstand abgesaugt und mit verdünnter Salzsäure und Wasser gewaschen. Getrocknet erhält man 49,4 g ockerfarbenes Pulver vom Rohschmelzpunkt 233 bis 236°. 49,3 g dieser o - Nitroacylamino - Verbindung werden in 450 ml DMF in Gegenwart von 6,0 g Raneyl-Nickel bei 50° mit Wasserstoff reduziert. Nach Absaugen des Katalysators engt man das Filtrat bis fast zur Trockne ein, nimmt mit 150 ml Eisessig auf und kocht 3 Stunden am Rückfluß, wobei der Benzimidazol-Ringschluß stattfindet. Das gebildete Benzimidazol wird durch Zugabe von 100 g Wasser ausgefällt (34 g). Durch mehrmalige Umkristallisation aus Dichloräthan und Acetonitril wird die Verbindung 110 als farbloses Pulver vom Schmp. 317—318° erhalten.

Beispiel 3

*Herstellung von 1,4 - Bis - [5 - trifluormethyl - 1 - äthyl - benzimidazolyl - (2)] - naphthalin (Verb. 111)*

Zunächst stellt man aus 4 - Chlor - 3 - nitrobenzotrifluorid und Äthylamin 3 - Nitro - 4 - äthylamino - benzotrifluorid (nach DOS 2 018 232, Beispiel 1) her und reduziert dieses katalytisch in Äthanol zum 3 - Amino - 4 - äthyl - amino - benzotrifluorid (farblose Kristalle vom Schmp. 72 bis 74°).

42,5 g dieser Diamino-Verbindung werden unter Stickstoff mit 26 ml Dimethylanilin und 100 ml Dioxan gelöst, mit einer Lösung 25,3 g Naphthalin - 1,4 - dicarbonsäurechlorid in 80 ml Toluol bie 25 bis 45° versetzt und zwei Stunden bei 70° nachgerührt. Nach Wasserdampfdestillation wird das abgeschiedene Produkt abgesaugt, mit Wasser gewaschen und getrocknet.

Die erhaltene Acylamino-Verbindung (56,7 g) wird 2 Stunden mit 150 ml Eisessig bei 118° gerührt. Durch Einrühren in Wasser können 51 g Rohprodukt ausgefällt werden. Die Verbindung (111) läßt sich durch Umkristallisation aus 200 ml DMF in farblosen Kristallen rein erhalten. Schmp. 297 bis 298°.

Für die Applikation auf Textilmaterial wurden je 100 mg dieser Aufheller in 10 ml Dimethylformamid in der Wärme gelöst und mit 5 ml eines Emulgators versetzt. Diese klare Lösung wurde unter Rühren in 85 ml Wasser gegeben. Es entsteht so eine stabile Dispersion, die 1 g/l Aufheller enthält.

Anwendungsbeispiel 1

Mit der vorher beschriebenen Dispersion, die 1 g/l der Verbindung 105 enthält, wurde ein Polyester-Gewebe getränkt und zwischen 2 Gummiwalzen auf eine Restfeuchte von 70% abgequetscht. Danach wurde die Probe in einem Spannrahmen bei 190°C während 30 Sekunden getrocknet und fixiert. Danach wies das Gewebe einen gegenüber einem nicht behandelten Gewebe wesentlich weißeren Eindruck auf.

Anwendungsbeispiel 2

Von einer in der vorher beschriebenen Weise hergestellten Dispersion, die 1 g/l der Verbindung 102 enthielt, wurde eine solche Menge abgemessen, daß die Aufhellermenge, berechnet auf das Substrat, 0,1% des Gewichtes ausmachte. Diese Menge wurde zu einer wäßrigen Flotte gegeben, die 2 g/l eines Bleichmittels enthielt. Ein Gewebe aus Perlon-Taft wurde in dieser Flotte in einem Verhältnis von 1:15 während 60 Minuten bei 98°C behandelt. Nach dem Spülen und Trocknen wies das Gewebe einen deutlich höheren Weißgrad als ein nicht behandeltes Gewebe auf.

Anwendungsbeispiel 3

Von der oben beschriebenen Dispersion der Verbindung 110 wurde eine Menge abgemessen, die einen Einsatz von 0,1%, bezogen auf ein Polyacrylnitril-Gewebe, entsprach. Diese Menge wurde in eine wäßrige Flotte gegeben, die 2 g/l $NaClO_2$ und 1 g/l eines Stabilisators enthielt. Diese Flotte wurde mit Essigsäure auf pH 3,5 eingestellt. Das Polyacrylnitrilgewebe wurde mit dieser Flotte während 1 Stunde bei 98°C im Flottenverhältnis 1:15 behandelt. Nach dem Spülen und Trocknen des Gewebes konnte ein beträchtlicher Weißgradanstieg beobachtet werden.

Anwendungsbeispiel 4

30 mg der Verbindung 105 wurden in 25 g Weichmachers und 1,5 g eines Stabilisators gelöst. Diese Mischung wurde unter Rühren zu 75 g eines Stammansatzes folgender Zusammensetzung gegeben:

75 g Polyvinylchlorid
2 g Titandioxid
0,2 g Wachs.

Diese Mischung wurde 10 Minuten lang auf dem Walzenstuhl bei 160°C ausgewalzt. Das erhaltene Walzfell ergab Weißgrade von 128 (Berger) bzw. 129 (Stensby). Bei Verwendung von 30 mg der Verbindung 102 wurden die Weißgrade 118 (Berger) und 121 (Stensby) gemessen.

Anwendungsbeispiel 5

Zu 100 g eines Polystyrol Granulats wurden 1,5 g Titandioxid und 0,03 g der Verbindung 105 zugegeben und unter ständigem Rühren gleichmäßig auf der Oberfläche des Granulats verteilt. Aus dem so behandelten Granulat wurden Spritzlinge hergestellt, deren Weißgrad mit 126 (Berger) und 132 (Stensby) bestimmt wurde. Bei Spritzlingen der gleichen Zusammensetzung, aber mit einem Gehalt an 0,03% der Verbindung 102 wurden folgende Weißgrade bestimmt:
115 (Berger); 124 (Stensby).

Anwendungsbeispiel 6

In gleicher Weise wie bei dem Anwendungsbeispiel 5 wurden Spritzlinge hergestellt aus Polypropylen Granulat mit einem Gehalt von 0,03% des Aufhellers 105 bzw. des Aufhellers 102. Es wurden folgende Weißgrade gemessen:

Aufheller 105 = 110 (Berger); 122 (Stensby)
Aufheller 102 = 113 (Berger); 124 (Stensby)

In gleicher Weise wurden Spritzlinge hergestellt aus Polyester, das bereits Titandioxid enthielt und 0,03% der Aufheller 105 bzw. 102.

Es wurden folgende Weißgrade ermittelt:

Aufheller 105 = 155 (Berger); 151 (Stensby)
Aufheller 102 = 152 (Berger); 152 (Stensby)

**Patentansprüche**

1. Verbindungen der allgemeinen Formel

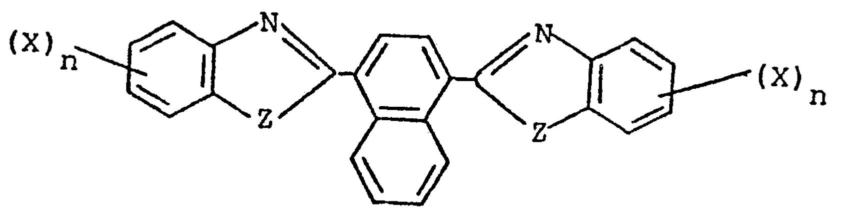

I

in welcher X eine Trifluormethylgruppe oder ein Fluoratom n 1 oder 2, Z ein Sauerstoffatom oder eine NR-Gruppierung bedeutet, in der R Wasserstoff oder eine $C_1$—$C_4$-Alkylgruppe ist, wobei die Benzoringe durch ein oder zwei $C_1$—$C_4$-Alkyl-, $C_1$—$C_4$-Alkoxy-, Halogen-, Cyan-, Carbo-($C_1$—$C_4$)-alkoxy-, Phenyl- oder Benzylgruppen substituiert sein können.

2. Verbindungen der allgemeinen Formel

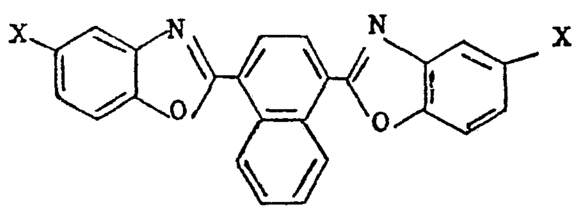

II

in welcher X eine Trifluormethylgruppe oder ein Fluoratom bedeutet.

3. Verbindungen der allgemeinen Formel

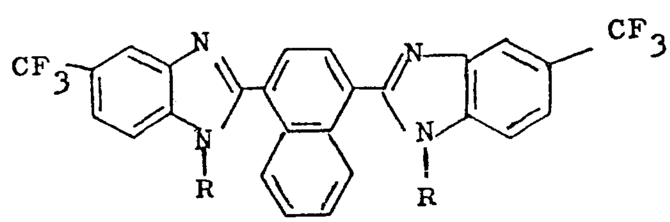

III

in welcher R Wasserstoff oder eine niedere Alkylgruppe bedeutet.

4. Verfahren zur Herstellung von Verbindungen nach Anspruch 1—3, dadurch gekennzeichnet, daß man 1 Mol des Dichlorids der 1,4 - Naphthalin - dicarbonsäure (IV) mit wenigstens 2 Mol eines o-Aminophenols der allgemeinen Formel

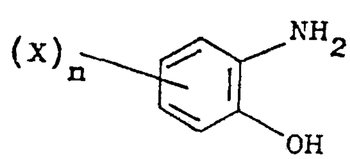

V

oder eines o-Nitroanilins der allgemeinen Formel

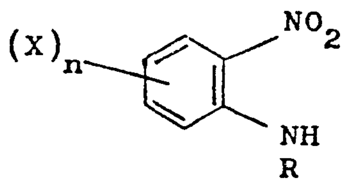

VII

umsetzt und die erhaltenen Acylamino-Verbindungen der allgemeinen Formel

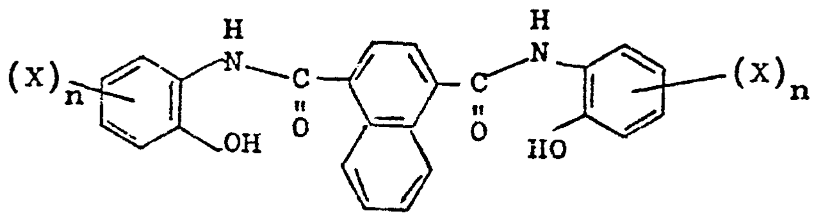

VI

in einer inerten Gasatmosphäre in hochsiedenden Lösungsmitteln, ggf. in Gegenwart von sauren Katalysatoren cyclisiert oder die erhaltenen Bis - o - nitro - acylamino - Verbindungen der allgemeinen Formel

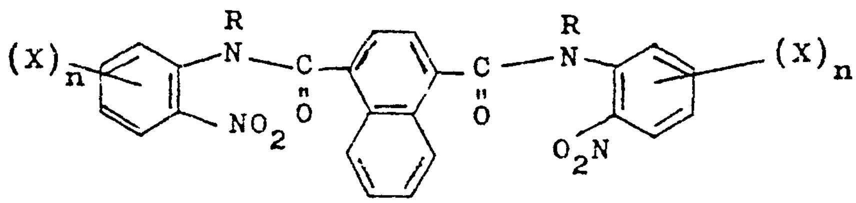

VIII

in einem Lösungsmittel mit Wasserstoff katalytisch oder einem anderen Reduktionsmittel reduziert und schließlich der erhaltenen Bis - o - amino - acylamino - Verbindungen durch Einwirkung saurer Reagentien cyclisiert.

5. Verwendung der Verbindungen gemäß Anspruch 1 bis 3 als optische Aufheller.

## Claims

1. Compounds of the general formula

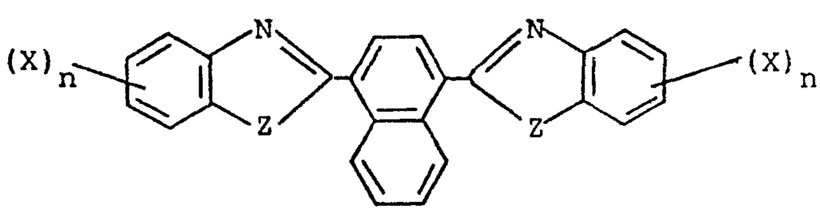

I

in which X is a trifluoromethyl group of a fluorine atom, n is 1 or 2, Z is an oxygen atom or a NR grouping, in which R represents hydrogen or a $C_1$—$C_4$-alkyl group, the benzo rings may be substituted by one or two $C_1$—$C_4$-alkyl-, $C_1$—$C_4$-alkoxy-, halogen-, cyano-, carbo-($C_1$—$C_4$)-alkoxy-, phenyl- or benzyl groups.

2. Compounds of the general formula

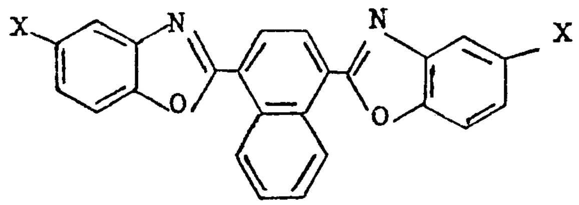

II

in which X is a trifluoromethyl group or a fluorine atom.

3. Compounds of the general formula

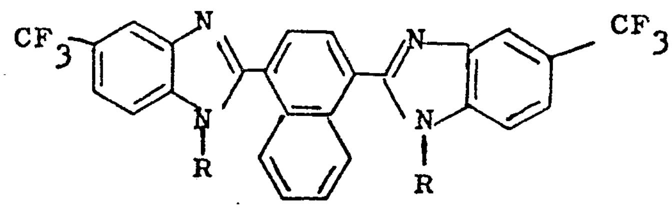

III

in which R is hydrogen or a lower alkyl group.

4. Process for the manufacture of compounds as claimed in claims 1 to 3, which comprises reacting 1 mol of the dichloride of 1,4 - naphthalene - dicarboxylic acid (IV) with at least 2 mols of an o-aminophenol of the general formula

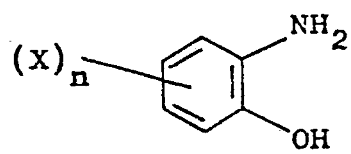

V

or of an o-nitroaniline of the general formula

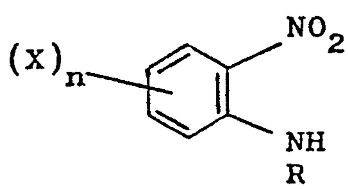

VII

and cyclizing the acylamino compounds obtained, which correspond to the general formula

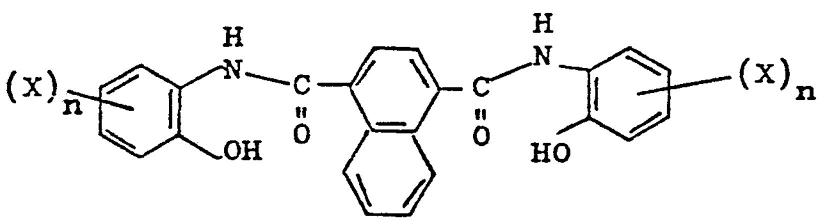

VI

in an inert gas atmosphere in high-boiling solvents, optionally in the presence of acid catalysts or reducing the bis - o - nitro-acylamino compounds obtained of the general formula

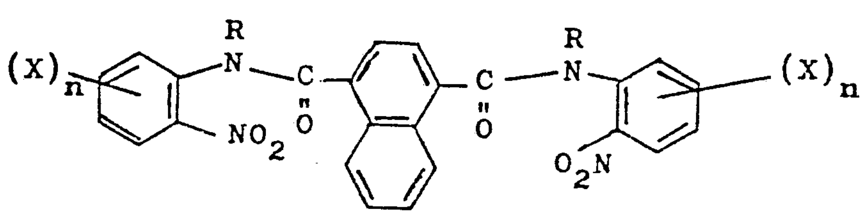

VIII

in a solvent with hydrogen by means of catalysts or with another reducing agent, and cyclizing subsequently, the bis - o - aminoacylamino compounds obtained by the action of acidic reagtents.

5. The use of the compounds as claimed in claims 1 to 3 as optical brighteners.

**Revendications**

1. Composés de formule générale

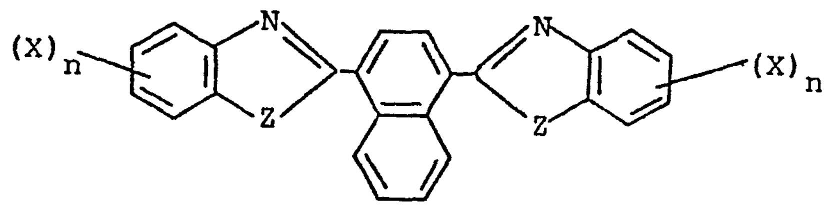

I

dans laquelle le symbole X représente un groupe trifluorométhyle ou un atome de fluor, n le nombre 1 ou 2 et Z un atome d'oxygène ou un groupe NR, R étant l'hydrogène ou un alkyle en $C_1$—$C_4$, et les cycles benzéniques pouvant être substitués par un ou deux alkyles ou alcoxy en $C_1$—$C_4$, atomes d'halogènes ou groupes cyano, ($C_1$—$C_4$) alcoxycarbonyles, phényles ou benzyles.

2. Composés selon la revendication 1, de formule générale

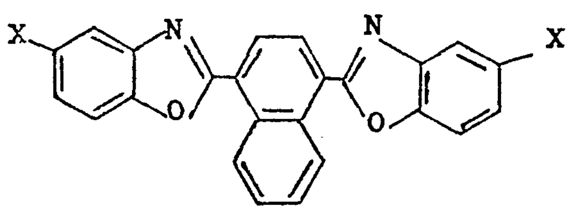

II

X désignant un groupe trifluorométhyle ou un atome de fluor.

3. Composés selon la revendication 1, de formule générale

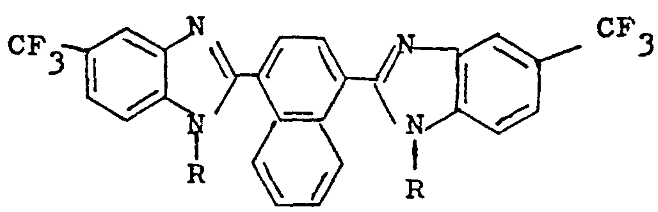

III

R désignant l'hydrogène ou un alkyle inférieur.

4. Procédé de préparation des composés selon l'une quelconque des revendications 1 à 3, caractérisé en ce que l'on fait réagir 1 mole du dichlorure de l'acide 1,4 - naphthalène - dicarboxylique (IV) avec au moins 2 moles d'un o-aminophénol de formule générale

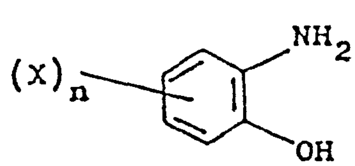

V

ou d'une o-nitroaniline de formule générale

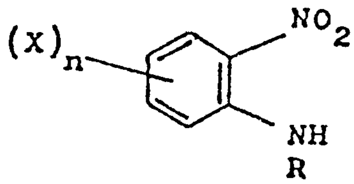

VII

et on cyclise le composé acylaminé ainsi formé, de formule générale

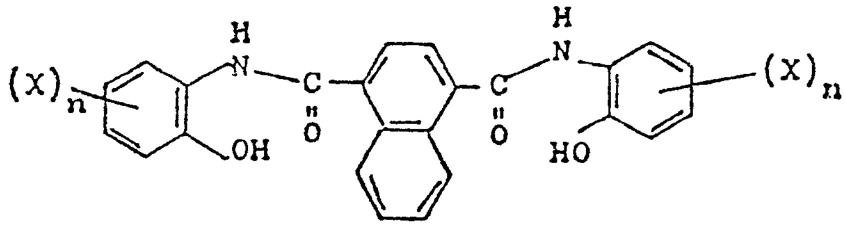

VI

sous une atmosphère de gaz inerte, dans un solvant à haut point d'ébullition, éventuellement en présence d'un catalyseur acide, ou bien on réduit catalytiquement par l'hydrogène, ou avec un autre agent réducteur, dans un solvant, le composé bis - o - nitroacylaminé formé, de formule générale

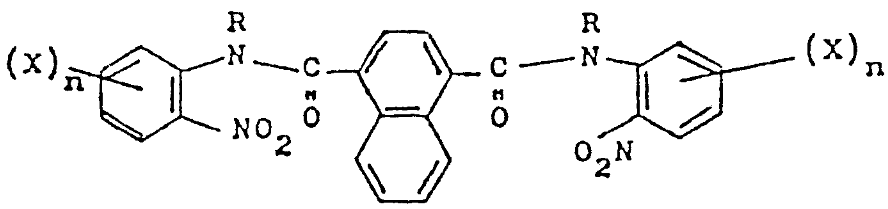

VIII

puis on cyclise le composé bis - o - amino - acylaminé ainsi obtenu sous l'action d'un réactif acide.

5. L'utilisation comme agents d'azurage optique des composés selon l'une quelconque des revendications 1 à 3.